# EUROPÄISCHE PATENTSCHRIFT

(11) **EP 1 946 638 B1**
(45) Veröffentlichungstag und Bekanntmachung des Hinweises auf die Patenterteilung: **16.08.2017**
(21) Anmeldenummer: 08000460.9
(22) Anmeldetag: 11.01.2008
(51) Int. Cl.: A01N 25/34, A01N 25/10, A01N 25/02, A01N 59/20, A01N 59/16, A01N 55/02, A01N 43/80, A01N 43/52, A01P 1/00, A61L 2/16, B60H 1/24, B60H 3/06, F24F 5/00, F24F 13/28, B01D 37/02, C09D 5/14, F28F 19/04, F28D 1/053, B01D 46/00, B60H 3/00

(54) **Verfahren zum Behandeln eines Bauteils mit einem Biozid**
Method for treating a component with a biocide
Procédé destiné au traitement d'un composant à l'aide d'un biocide

(30) Priorität: 17.01.2007 DE 102007003322
(43) Veröffentlichungstag der Anmeldung: 23.07.2008
(73) Patentinhaber: MAHLE Behr GmbH & Co. KG, 70469 Stuttgart (DE)
(72) Erfinder: Boger, Snjezana, 73734 Esslingen (DE); Burr, Reinhold, 89518 Heidenheim (DE); Mamber, Oliver, 71706 Markgröningen (DE); Schaper, Jörg, 70806 Kornwestheim (DE); Wolf, Walter, 71570 Oppenweiler-Zell (DE)
(74) Vertreter: Grauel, Andreas

(56) Entgegenhaltungen:
- FR-A1- 2 720 340
- US-A- 5 366 004
- US-A- 5 885 605

## Beschreibung

Die Erfindung betrifft ein Verfahren zum Behandeln eines Bauteils mit einem Biozid, für eine Belüftungs-, Heizungs- und/oder Klimaanlage eines Fahrzeugs, gemäß dem Oberbegriff des Anspruches 1.

Aus der DE 102 25 324 A1 ist eine antimikrobielle Beschichtung für Bauteile bekannt, wobei der Beschichtungswerkstoff ein Bakterienwachstum auf den beschichteten Flächen teilweise oder vollständig unterdrückt. Der antimikrobielle Lack enthält hierbei Nanoteilchen mit einer Teilchengröße kleiner 100 nm und die Oberfläche der Nanoteilchen ist mit Silberionen oder Kupferionen oder elementarem Silber oder elementarem Kupfer angereichert. Der Lack weist ein nanoskaliges, keramikbildendes Pulver auf, aus der Gruppe der Oxyd-, Sulfid-, Carbid- oder Nitridpulver. Als Grundwerkstoffe für das Aufbringen einer derartigen antimikrobiellen Schicht eignen sich alle Arten von Kunststoffen und Metallen, sowie Holz, Email und Keramiken.

Die EP 0 409 130 B1 offenbart einen Wärmetauscher aus Aluminium, der eine Anzahl von Rohren, die von einem Wärmetauscherfluid durchströmt werden, aus Aluminium und zwischen den Rohren angeordnete Lamellen aus Aluminium auf. Hierbei ist ein erster Schutzfilm eines chemischen Umwandlungsüberzugs, gebildet auf den Metalloberflächen der Rohre und Lamellen und enthaltend eine Metallkomponente, und ein auf dem ersten Schutzfilm angeordneter zweiter Schutzfilm zumindest auf den Lamellen vorhanden, der ein antimikrobielles Mittel, insbesondere 2,2'-Dithiobis-(pyridin-1-oxid) enthält. Der zweite Schutzfilm ist hierbei hitzebeständig bis etwa 220°C, einheitlich löslich in einer sauren Lösung bzw. in einer Säurelösung und wird unlöslich. Der zweite Schutzfilm wird auf dem ersten Schutzfilm durch ein mehrwertiges Metallion abgesetzt. Ferner besteht eine chemische Bindung zwischen dem zweiten Schutzfilm und der Metallkomponente des ersten Schutzfilms. Der zweite Schutzfilm umfasst bevorzugt einen Harzüberzug aus einem organischen Polymer.

Weitere Beschichtungen, welche die Ansiedlung von Mikroorganismen auf Oberflächen verhindern, sind beispielsweise aus der DE 197 50 122 A1 oder der DE 100 45 605 A1 bekannt.

Ferner ist aus der DE 199 10 356 A1 bekannt, Luft unter Zugabe bzw. Aufrechterhaltung der Wirkung eines biologisch völlig untoxischen Mittels in einem stark verkeimungsarmen Zustand zu halten.

Aus der Patentschrift US 5 366 004 ist ein selbst desinfizierendes Bauteil gemäß dem Oberbegriff des vorliegenden Anspruchs 1 bekannt.

Beschichtungen mit biozidhaltigen Materialien aber auch ein direktes Beimischen eines Mittels in die einem Raum zuzuführende Luft lassen noch Wünsche offen.

Es ist Aufgabe der Erfindung, einen verbesserten Schutz mit Hilfe eines Biozids zur Verfügung zu stellen.

Diese Aufgabe wird gelöst durch ein Verfahren mit den Merkmalen des Anspruchs 1. Vorteilhafte Ausgestaltungen sind Gegenstand der Unteransprüche.

Die Erfindung betrifft ein Verfahren zum Behandeln eines Bauteils mit einem Biozid, wobei das Bauteil und/oder ein weiteres Bauteil, wie beispielsweise ein das erste Bauteil umgebende Gehäuse ein biozidhaltiges Material aufweist, welches an einem ersten Bereich wirken soll. Hierbei ist das biozidhaltige Material in einem zweiten Bereich des Bauteils und/oder einem Bereich des weiteren Bauteils angeordnet, welcher beabstandet vom eigentlichen Einsatzort des Biozids, also dem ersten Bereich, angeordnet ist. Das Biozid wird allmählich mittels eines Trägermediums aus dem Material des zweiten Bereichs herausgelöst und anschließend wird das biozidhaltige Trägermedium zum ersten Bereich gefördert, welcher den Einsatzort für das Biozid bildet.

Die Förderung des biozidhaltigen Trägermediums erfolgt bevorzugt schwerkraftbetätigt. Alternativ kann das biozidhaltige Trägermedium auch auf beliebige andere Weise gefördert werden, insbesondere bevorzugt luftströmungsbetätigt und/oder zentrifugalkraftbetätigt.

Beim Trägermedium handelt es sich bevorzugt um Wasser, insbesondere Kondenswasser, Spritzwasser oder Regenwasser, welches bspw. bei einem Fahrzeug regelmäßig vorkommt.

Für ein derartiges Verfahren ist insbesondere ein Bauteil vorgesehen, bei welchem in einem ersten Bereich ein Einsatzort für ein Biozid vorgesehen ist, und ferner ein beabstandet vom ersten Bereich angeordneter, zweiter Bereich vorgesehen ist, in welchem zumindest die Oberfläche des Bauteils zumindest teilweise durch herauslösbares Biozid gebildet ist, wobei das Biozid vom zweiten Bereich des Bauteils zum ersten Bereich des Bauteils mittels eines Trägermediums transportierbar ist.

Das Bauteil weist vorzugsweise zumindest bereichsweise ein Material mit einem Biozid auf, insbesondere in einer Außenbeschichtung einer Oberfläche oder eines Oberflächenbereichs des Bauteils, bei dem das Biozid während des Einsatzes oder Betriebs des Bauteils oder einer Vorrichtung in welcher das Bauteil verbaut ist, aus dem Material herauslösbar ist, wobei das Biozid durch ein Fluid, insbesondere Wasser, insbesondere bevorzugt in der Form von Kondenswasser, Spritzwasser oder Regenwasser, das zumindest kurzzeitig im Bereich der Oberfläche eines biozidhaltigen Bereichs vorhanden ist, aus dem Material herausgelöst wird. Unter einer Vorrichtung in die ein entsprechendes Bauteil eingesetzt ist, ist eine Belüftungs-, Heizungs- und/oder Klimaanlage eines Fahrzeugs zu verstehen, wobei sich der Betrieb auf diese Vorrichtung oder allgemeiner auf das Fahrzeug selbst bezieht. Dadurch, dass das Biozid aus dem biozidhaltigen Material allmählich durch das Fluid herauslösbar ist, kann über einen im Wesentlichen abschätz- oder berechenbaren Zeitraum hinweg ausreichend Biozid zur Verhinderung beispielsweise eines Mikrobenbewuchses in das Material depotartig eingelagert werden. Das Biozid kann bedarfsgerecht zusammengestellt sein, wobei es durch eine beliebige Kombination von Wirkstoffen, beispielsweise Algiziden, Bakteriziden, Fungiziden, Mikrobiziden, Viruziden oder Rodentiziden gebildet sein kann. Durch das Vorsehen eines Biozids können insbesondere unangenehme Gerüche und die Bildung von Biofilmen auf Oberflächen verhindert werden, so dass insbesondere bei Klimaanlagen der Komfort steigt und die Kosten für Reinigungen/Desinfektionen verringert werden können. Als Biozide sind insbesondere folgende Wirkstoffe geeignet: Silber, insbesondere in der Form von Nanopartikeln, Silbersalze, Kupfer, insbesondere in der Form von Nanopartikeln, Kupfersalze, Natriumpyrithion, Zinkpyrithion, Benzimidazole (z.B. Thiabendazol), Octylisothiazolon, Dichloroctylisothiazolon, quartäre Ammoniumsalze und Chlormethylisothiazolinon. Der Biozidgehalt des biozidhaltigen Materials beträgt vorzugsweise 0,1 bis 20 Gew.-%, insbesondere bevorzugt 0,5 bis 15 Gew.-% und besonders bevorzugt 1 bis bis 10 Gew.-%. Hierbei kann der Biozidgehalt auch über die Oberfläche und/oder den Abstand zur Oberfläche des Bauteils variieren, so dass eine gezielte Freisetzung des oder der Wirkstoffe möglich ist. Ebenfalls kann das Biozid eingekapselt sein.

Besonders bevorzugt ist das in geringen Mengen herausgelöste Biozid gemeinsam mit dem im biozidhaltigen Bereich sich sammelnden oder gegebenenfalls demselben zugeführten Fluid zu einem anderen Bereich des Bauteils führbar, welcher durch ein biozidfreies Material gebildet ist. Die Weiterleitung des mit dem Biozid angereicherten Fluids kann auf besonders einfache Weise mittels Schwerkraft, mitgerissen durch Luft, welche das Bauteil um- oder durchströmt, oder mittels Zentrifugalkraft erfolgen. Dies ermöglicht, dass auch ein Bereich, in dem kein Biozid eingelagert ist, frei von Mikroorganismen gehalten werden kann. Besonders vorteilhaft ist der Transport des biozidhaltigen Fluids von einem biozidhaltigen Bereich zu einem anderen, nicht biozidhaltigen Bereich, beispielsweise im Falle von Wellrippen eines Verdampfers, in deren Bereich das Auftragen eines Biozids, beispielsweise enthalten in einem Polymer, zu Wirkungsgradeinbussen führen würde.

Das Bauteil, welches ein biozidhaltiges Material aufweist, ist ein Verdampfer wobei das Biozid im Material der Sammelkästen des Verdampfers angeordnet ist. Durch das sich im Bereich des oberen Sammelkastens sammelnde Kondenswasser wird das Biozid aus dem biozidhaltigen Materal herausgelöst oder durch langsames Auflösen der Schicht freigesetzt, und über die Wärmeübertragungsfläche, d.h. das Netz des Verdampfers, nach unten gefördert, wobei die Wärmeübertragungsfläche automatisch mit dem Wirkstoff behandelt wird. Durch das im Bereich des unteren Sammelkastens vorhandene Biozid wird hingegen insbesondere das sich unten sammelnde Kondenswasser behandelt, also entkeimt.

Beim Material, das mit einem Biozid versetzt ist, handelt es sich bevorzugt um einen Kunststoff, insbesondere bevorzugt um ein Polymermaterial, wie PET, PA6, PA6.6, PP oder PE. Das Biozid kann beispielsweise während des Aufschmelzen des Granulats in einem Reaktor oder einem Extruder dem Kunststoff beigemischt werden. Auch eine nachträgliche Einbringung des Biozids mittels Diffusion, beispielsweise aus einer Lösung, ist möglich. Eine Eindiffusion des Wirkstoffes kann beispielsweise im Falle der Verwendung bei Filterfasern sowohl auf die (fertig) gesponnene Faser als auch auf das Ausgangsgranulat angewandt werden. Entsprechendes gilt auch für das Spritzgießverfahren, d.h. eine Eindiffusion ist sowohl in das Granulat als auch das fertig gegossene Werkstück möglich. Der Wirkstoff kann ferner nachträglich auf die Oberfläche in Form einer Beschichtung aufgebracht werden. Auch im Falle von Fasern ist die Einbringung des Biozids in verkapselter Form möglich, so dass der Wirkstoff zeitversetzt aus den Kapseln freigesetzt werden kann. Neben der Beimengung von Bioziden, beispielsweise in Form der weiter oben angegebenen Substanzen, können auch durch eine Copolymerisation biozid wirksame Polymere eingestellt werden, wie beispielsweise organische Phosphorigsäureester als Copolymer für Polyester, Polystyrolfasern mit einer Dimethylaminomethylengruppe am Phenylring.

Insbesondere bevorzugt löst sich das biozidhaltige Material allmählich von außen her auf, d.h. die Materialdicke nimmt über die Benutzungsdauer hin allmählich ab, so dass stets neue Bereiche des biozidhaltigen Materials an die Oberfläche gelangen. Alternativ zu einem Auflösen des biozidhaltigen Materials kann auch eine Wasserpermeation vorgesehen sein, welche ein Herauslösen der Wirkstoffe ermöglicht. Ferner ist eine Einlagerung der Wirkstoffe in verkapselter Form möglich, wobei die Kapseln zeitlich versetzt freigegeben werden.

Die Oberfläche der biozidhaltigen Beschichtung oder des biozidhaltigen Materials kann geschlossen- oder offenporig sein. Ebenfalls sind waben- oder schaumartige Strukturen zur Vergrößerung der Oberfläche möglich. Ferner kann die Oberfläche fest vernetzt oder plastisch oder elastisch verformbar ausgebildet sein.

Handelt es sich beim Bauteil um einen beschichteten Verdampfer, so ist besonders bevorzugt die Beschichtung mit der biozidhaltigen Schicht ausschließlich im Bereich des oder der Sammelkästen des Verdampfers, insbesondere bevorzugt des oberen Sammelkastens, vorgesehen. Alternativ können der oder die Sammelkästen auch bereichsweise - insbesondere bevorzugt mit Abstand zu Aluminiumbauteilen, wie Flachrohren oder Wellrippen - oder gegebenenfalls auch vollständig aus einem biozidhaltigen Kunststoff geformt sein. Die Herstellung kann bevorzugt mittels Spritzgießens, insbesondere mittels eines Zweikomponenten-Spritzgießverfahrens, erfolgen. Im Falle einer nachträglich aufgebrachten Beschichtung kann dieselbe auf beliebige Weise aufgebracht werden, beispielsweise mittels Tauchens, Flutens, Besprühens, Aufklebens, Aufsteckens, Aufschmelzens, eines CVD- oder PVD-Verfahrens.

Es handelt sich beim Bauteil um einen Verdampfer, welcher mindestens einen im in ein Fahrzeug. eingebauten Zustand oben angeordneten Sammelkasten aufweist. Dieser Sammelkasten ist aus Kunststoff hergestellt, insbesondere spritzgegossen, wobei er vorzugsweise 0,1 bis 20 Gew.-%, insbesondere bevorzugt 0,5 bis 10 Gew.-% Biozid enthält. Der Sammelkasten wird mit dem Wärmeübertragungsbereich (Wärmetauschernetz) auf bekannte Weise verbunden, so dass ein direktes Überströmen des biozidhaltigen Trägermediums vom Sammelkasten zum Wärmeübertragungsbereich möglich ist.

Die Anbringung eines biozidhaltigen Kunststoffbauteils, wobei das Bauteil vorzugsweise 0,1 bis 20 Gew.-%, insbesondere bevorzugt 0,5 bis 10 Gew.-% Biozid enthält, an einem Verdampfer erfolgt insbesondere bevorzugt nach dem Verlöten des Wärmetauschernetzes mittels Clipsens, Schraubens, Klebens, Schweißens oder Nietens. Hierbei bildet das Kunststoffbauteil ein Bioziddepot im Bereich des Verdampfers.

Besonders bevorzugt weist das Bauteil zumindest einen ersten Bereich, in welchem sich Kondenswasser und/oder Spritzwasser und/oder Regenwasser sammelt oder bildet und einen zweiten Bereich auf, wobei das Wasser nach unten über den anderen, zweiten Bereich des Bauteils abläuft. Hierbei ist zumindest der erste Bereich mit dem biozidhaltigen Kunststoff ausgebildet oder versehen. Insbesondere bevorzugt ist der andere, zweite Bereich nicht aus einem biozidhaltigen Material gebildet, soll aber beispielsweise vor einem Mikrobenbewuchs mit Hilfe des Biozids geschützt werden.

Das Biozid kann faserförmig sein und/oder in der Form von Mikro- und/oder Nanopartikeln vorliegen. Insbesondere bevorzugt betragen die Durchmesser der Partikel oder Fasern wenige 1/10-tel Millimeter, insbesondere bevorzugt 10-100 nm.

Im Folgenden wird die Erfindung anhand von mehreren Ausführungsbeispielen, teilweise unter Bezugnahme auf die Zeichnung, im Einzelnen erläutert. Es zeigen:
- Fig. 1: eine schematische Darstellung eines Wärmetauschers, und
- Fig. 2: einen stark schematisierten, ausschnittsweisen Schnitt senkrecht zu einer mit einer Beschichtung versehenen Oberfläche.

Ein Verdampfer 1, der Teil einer Kraftfahrzeug-Klimaanlage ist, weist zwei Sammelkästen 2, wobei der erste Sammelkasten 2 oberhalb des anderen Sammelkastens 2 angeordnet ist, dazwischen angeordnete kältemittelführende Flachrohre 3 und der Vergrößerung der Wärmeübertragungsfläche dienende Wellrippen 4 auf. Die Kältemittelzu- bzw. -ableitung erfolgt mittels Anschlussleitungen 5.

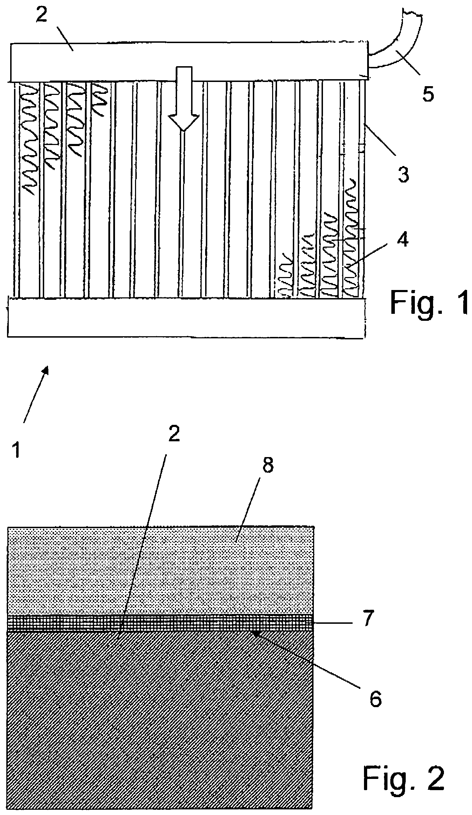

Der Verdampfer 1 ist vorliegend auf an sich bekannte Weise aus Aluminium hergestellt, wobei die einzelnen Bauteile miteinander verlötet sind. Die (Aluminium-)Oberfläche 6 des Verdampfers 1 ist vorliegend, wie in Fig. 2 schematisch angedeutet, durchgehend mit einer Konversionsschicht 7 zwecks Korrosionsschutzes versehen. Ferner ist im Bereich des oberen Sammelkastens 2 eine 2000 µm dicke Polymerbeschichtung 8 vorgesehen, in welcher ein Biozid enthalten ist. Hierbei besteht ein Gewichtsverhältnis von Polymer zu Biozid von 10:1. Das Biozid selbst wird durch eine Mischung verschiedener chemisch und/oder biologisch wirksamer Substanzen, wie unter anderem nanoskalige Silberpartikel, gebildet, wobei als Substanzen in Bezug auf ihre Wirkung vorliegend ein Algizid, ein Bakterizid, ein Fungizid und/oder ein Mikrobizid Bestandteil des Biozids sind. Dabei ist das Biozid depotartig in der Beschichtung enthalten, d.h. es wird nur allmählich und relativ gleichmäßig abgegeben, wofür sich die Beschichtung langsam bei Kontakt mit dem sich sammelnden Kondenswasser auflöst (hydrolysiert wird), wobei die wirksamen Substanzen freigegeben werden.

Sammelt sich Kondenswasser am oberen Sammelkasten, so löst dieses langsam Biozid aus der Beschichtung. Wenn die Kondenswassertropfen zu groß werden, fließen sie in Folge der Schwerkraft langsam nach unten ab, wodurch das mit Biozid angereicherte Kondenswasser in einen Bereich (Wärmetauschernetz) des Verdampfers 1 gelangt, in welchem ausschließlich besagte Konversionsschicht 7 und keine Polymerbeschichtung vorgesehen ist, d.h. das Biozid wird aus einem ersten Bereich (oberer Sammelkasten 2) in einen zweiten Bereich (Wärmeübertragungsfläche) transportiert, in welchem die Wirkung des Biozids gewünscht ist, aber beispielsweise aus herstellungstechnischen Gründen keine Beschichtung mit einem Biozid vorgesehen ist. Dieser Transportvorgang ist in Fig. 1 durch einen Pfeil angedeutet.

Alternativ zu einem Wärmetauscher in Flachrohrbauweise ist eine entsprechende Ausgestaltung beispielsweise auch bei einem Wärmetauscher in Scheibenbauweise oder einem anderen Wärmetauscher mit mindestens einem obenliegenden Sammelkasten möglich.

Gemäß der beanspruchten nicht in der Zeichnung dargestellten Erfindung ist keine Beschichtung mit einem ein Biozid enthaltenden Material vorgesehen, sondern das ein Biozid enthaltende Material selbst bildet einen Teil des Sammelkasten eines Verdampfers. Hierfür sind Nanopartikel, insbesondere Silbernanopartikel, in einem Granulat enthalten, welches für die Herstellung eines Sammelkastens mittels Spritzgießens verwendet wird. Auch in diesem Fall werden die wirksamen Substanzen allmählich durch das Kondenswasser aus dem Material herausgelöst und werden vom Kondenswasser schwerkraftbetätigt in Richtung der unter dem oben angeordneten Sammelkasten angeordneten Wärmeübertragungsfläche transportiert, wo sie ihre Wirkung entfalten.

Gemäß einem dritten, nicht in der Zeichnung dargestellten Ausführungsbeispiel wird ein Sammelkasten mittels eines Zweikomponenten-Spritzgießverfahrens hergestellt, wobei der im gespritzten Zustand in Bereichen beabstandet von den Wellrippen und den Rohren (zwecks Vermeidung einer elektrochemischen Korrosion der Aluminiumbauteile des Wärmetauschers) außen angeordneten Komponente das Biozid in verkapselter Form im Bereich der Extruderschnecke der Spritzgießmaschine zugegeben wird. Hierbei kann prinzipiell für beide Komponenten das gleiche Basismaterial verwendet werden, wobei nur besagtem bereichsweise außen angeordneten Teil das Biozid beigemischt wird, bevorzugt wird jedoch für die biozidhaltige Außenschicht ein Basismaterial verwendet, welches ein Herauslösen des hierin enthaltenen Biozids ermöglicht. Als Basismaterial, insbesondere für die biozidfreie Grundkomponente, dient vorzugsweise ein Thermoplast, insbesondere ein Polyolefin, wie PP, PE, PA6 und/oder PA6.6. Auch in diesem Fall erfolgt ein Herauslösen des Biozids im Bereich des oberen Sammelkastens durch sich dort sammelndes Kondenswasser sowie ein Transport des biozidhaltigen Kondenswassers in Folge der Schwerkraft nach unten, in den Bereich des Wärmetauschernetzes.

Alternativ zu einer Schwerkraftförderung oder einer Mitnahme in einem Gas-, insbesondere Luftstrom, kann eine Förderung des aus einem ersten Bereich eines Bauteils mit Hilfe eines Trägermediums, insbesondere Wasser, herausgelösten Biozids auch zentrifugalkraftbetätigt erfolgen.

## Patentansprüche

1. Bauteil, bei welchem in einem ersten Bereich ein Einsatzort für ein Biozid und ein zweiter Bereich vorgesehen ist, in welchem zumindest die Oberfläche des Bauteils zumindest teilweise durch herauslösbares Biozid gebildet ist, wobei das Biozid vom zweiten Bereich des Bauteils zum ersten Bereich des Bauteils mittels eines Trägermediums transportierbar ist, wobei das Biozid gemeinsam mit dem im biozidhaltigen, zweiten Bereich vorhandenen Trägermedium zum ersten Bereich des Bauteils führbar ist, welcher durch ein biozidfreies Material gebildet ist und der zweite Bereich des Bauteils, in welchem das Biozid angeordnet ist, durch ein biozidhaltiges Polymer gebildet ist, wobei das Bauteil ein Verdampfer einer Belüftungs-, Heizungs- und/oder Klimaanlage eines Fahrzeugs ist **dadurch gekennzeichnet, dass** der oder die Sammelkästen des Verdampfers aus einem biozidhaltigen Kunststoff geformt sind, und das Wärmetauschernetz durch ein biozidfreies Material gebildet ist.

2. Bauteil nach Anspruch 1, **dadurch gekennzeichnet, dass** das Biozid Silber, insbesondere in der Form von Nanopartikeln, Silbersalz, Kupfer, insbesondere in der Form von Nanopartikeln, Kupfersalz, Natriumpyrithion, Zinkpyrithion, Benzimidazole, insbesondere Thiabendazol, Octylisothiazolon, Dichloroctylisothiazolon, ein quartäres Ammoniumsalz und/oder Chlormethylisothiazolinon ist.

3. Bauteil nach einem der Ansprüche 1 oder 2, **dadurch gekennzeichnet, dass** der Biozidgehalt des biozidhaltigen Materials 0,1 bis 20 Ges.-%, insbesondere 0,5 bis 15 Gew.-%, beträgt.

4. Belüftungs-, Heizungs- und/oder Klimaanlage, insbesondere eines Fahrzeugs, **dadurch gekennzeichnet, dass** die Belüftungs-, Heizungs- und/oder Klimaanlage ein Bauteil nach einem der vorhergehenden Ansprüche aufweist.

## Claims

1. A component, wherein in a first region an application area for a biocide and a second region in which at least the surface of the component is at least partially formed by detachable biocide are provided, wherein the biocide can be transported from the second region of the component to the first region of the component by means of a support medium, wherein the biocide together with the support medium present in the second, biocide-containing region can be guided to the first region of the component which is formed by a material free from biocide and the second region of the component, in which the biocide is arranged, is formed by a biocide-containing polymer, wherein the component is an evaporator of a ventilation, heating and/or air-conditioning system of a vehicle, **characterised in that** the one or more collecting tanks of the evaporator are formed from a biocide-containing plastic and the heat exchanger net is formed by a material free from biocide.

2. The component according to claim 1, **characterised in that** the biocide is silver, in particular in the form of nanoparticles, silver salt, copper, in particular in the form of nanoparticles, copper salt, sodium pyrithione, zinc pyrithione, benzimidazoles, in particular thiabendazole, octylisothiazolone, dichlorotylisothiazolone, a quaternary ammonium salt and/or chloromethylisothiazolinone.

3. The component according to one of claims 1 or 2, **characterised in that** the biocide content of the biocide-containing material is 0.1 to 20 % by weight, in particular 0.5 to 15 % by weight.

4. A ventilation, heating and/or air-conditioning system, in particular of a vehicle, **characterised in that** the ventilation, heating and/or air-conditioning system has a component according to one of the preceding claims.

## Revendications

1. Composant dans lequel il est prévu, dans une première zone, un lieu d'utilisation pour un biocide, et une seconde zone dans laquelle au moins la surface du composant est formée au moins partiellement par un biocide pouvant être éliminé, où le biocide peut être transporté, au moyen d'un milieu formant support, depuis la seconde zone du composant jusqu'à la première zone du composant, où le biocide, de façon conjointe avec le milieu formant support présent dans la seconde zone contenant un biocide, peut être guidé jusqu'à la première zone du composant, zone qui est formée par un matériau sans biocide, et la seconde zone du composant, zone dans laquelle le biocide est disposé, est formée par un polymère contenant un biocide, où le composant est un évaporateur d'un système de ventilation, de chauffage et / ou de climatisation d'un véhicule, **caractérisé en ce que** le ou les bac(s) collecteur(s) de l'évaporateur est ou sont formé(s) à partir d'une matière plastique contenant un biocide, et le réseau d'échangeurs de chaleur est formé par un matériau sans biocide.

2. Composant selon la revendication 1, **caractérisé en ce que** le biocide est de l'argent, en particulier se présentant sous la forme de nanoparticules, de sel d'argent, de cuivre, en particulier se présentant sous la forme de nanoparticules, de sel de cuivre, de pyrithione de sodium, de pyrithione de zinc, de benzimidazoles, en particulier sous la forme de thiabendazole, d'octylisothiazolinone, de dichloro-octylisothiazolinone, ledit biocide étant un sel d'ammonium quaternaire et / ou de la chloro-méthyl-isothiazolinone.

3. Composant selon l'une des revendications 1 ou 2, **caractérisé en ce que** la teneur en biocide du matériau contenant un biocide est comprise entre 0,1 % en poids et 20 % en poids, en particulier comprise entre 0,5 % en poids et 15 % en poids.

4. Système de ventilation, de chauffage et / ou de climatisation, en particulier d'un véhicule, **caractérisé en ce que** le système de ventilation, de chauffage et / ou de climatisation présente un composant selon l'une quelconque des revendications précédentes.
